(19) Europäisches Patentamt
European Patent Office
Office européen des brevets

(11) EP 0 616 035 B1

(12) **EUROPÄISCHE PATENTSCHRIFT**

(45) Veröffentlichungstag und Bekanntmachung des Hinweises auf die Patenterteilung:
**18.06.2003 Patentblatt 2003/25**

(51) Int Cl.[7]: **C12N 15/82**, C12N 15/56, C12N 15/29, A01H 5/00, A01N 63/00

(21) Anmeldenummer: **93116011.3**

(22) Anmeldetag: **04.10.1993**

(54) **Transgene Pilz-resistente Pflanzen**

Transgenic fungi resistant plants

Plantes transgéniques résistant aux champignons

(84) Benannte Vertragsstaaten:
**AT BE CH DE DK ES FR GB GR IE IT LI NL PT SE**

(30) Priorität: **09.10.1992 DE 4234131**

(43) Veröffentlichungstag der Anmeldung:
**21.09.1994 Patentblatt 1994/38**

(73) Patentinhaber: **Max-Planck-Gesellschaft zur Förderung der Wissenschaften e.V.
D-37073 Göttingen (DE)**

(72) Erfinder:
- **Logemann, Jürgen, Dr.
NL-2317 NB Leiden (NL)**
- **Jach, Guido
D-50678 Köln (DE)**
- **Görnhardt, Birgit
D-51145 Köln (DE)**
- **Mundy, John, Dr.
1760 V Copenhagen (DK)**
- **Schell, Jeff, Prof.
D-50829 Köln (DE)**
- **Eckes, Peter, Dr.
D-65779 Kelkheim (Taunus) (DE)**
- **Chet, Ilan, Prof.
Nes Ziona 70400 (IL)**

(74) Vertreter: **von Hellfeld, Axel, Dr. Dipl.-Phys.
Wuesthoff & Wuesthoff
Patent- und Rechtsanwälte
Schweigerstrasse 2
81541 München (DE)**

(56) Entgegenhaltungen:

| | |
|---|---|
| **EP-A- 0 440 304** | **WO-A-89/04371** |
| **WO-A-91/19738** | **WO-A-92/16632** |
| **WO-A-92/17591** | **WO-A-94/08009** |
| **US-A- 4 940 840** | **US-A- 4 970 168** |

- **DEV. PLANT PATHOL. 2(MECHANISMS OF PLANT DEFENSE RESPONSES), SYMP. HELD AUG. 24-27, 1992 Seite 449 BOJSEN, K., ET AL. 'Genetic transformation of Nicotiana benthamiana with chitinase and beta-1,3-glucanase genes from Beta vulgaris (sugar beet)'**
- **BIOTECHNOLOGY, Bd.10, Nr.3, März 1992, NEW YORK US Seiten 305 - 308 LOGEMANN, J., ET AL. 'Expression of a barley ribosome-inactivating protein leads to increased fungal protection in transgenic tobacco plants'**
- **JOURNAL OF BIOLOGICAL CHEMISTRY, Bd.266, Nr.3, 25. Januar 1991, BALTIMORE, MD US Seiten 1564 - 1573 LEAH, R., ET AL. 'Biochemical and molecular charcterization of three barley seed proteins and antifungal properties'**
- **BIOPRACTICE, Bd.1, 1992 Seiten 33 - 40 JACH, G., ET AL. 'Expression of a bacterial chitinase leads to improved resistance of transgenic tobacco plants against fungal infection'**
- **CURRENT PLANT SCIENCE AND BIOTECHNOLOGY IN AGRICULTURE, VOL.14. ADVANCES IN MOLECULAR GENETICS OF PLANT-MICROBE INTERACTIONS, VOL. 2. 6TH SYMPOSIUM ON MOLECULAR PLANT-MICROBE INTERACTIONS JULY, 1992. Seiten 567 - 571 DUNSMUIR, P., ET AL. 'Resistance to Rhizoctonia-solani in transgenic tobacco'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

- **SCIENCE, Bd.254, 1991 Seiten 1194 - 1197 BROGLIE, K., ET AL. 'Transgenic plants with enhanced resistance to the fungal pathogen Rhizoctonia solani'**

## Beschreibung

Die Erfindung betrifft eine Pilz-resistente Pflanze sowie ein Verfahren zu deren Erzeugung.

Im Stand der Technik ist bekannt, daß der Befall einer Pflanze durch Pathogene eine Reihe verschiedener Reaktionen hervorgerufen werden. Dazu gehören zum Beispiel Veränderungen in der Zellwandstruktur, die Synthese antimikrobiell wirkender Phytoalexine, die Akkumulation von sogenannten PR-Proteinen ("Pathogenesis-related"), Protease-Inhibitoren und Enzyme mit hydrolytischen Funktionen (Hahlbrock und Grisebach in Ann. Rev. Plant. Physiol., 30, (1979), 105-130).

Viele Pathogene (Pilze und Insekten) weisen als Bestandteil ihrer Zellwand Chitin auf. Demgegenüber besitzen Plfanzen kein Chitin. Es ist nun in einigen Fällen nachgewiesen worden, daß Plfanzen nach einem pathogenen Befall verstärkt Chitinasen produzieren. Chitinasen gehören zu den Enzymen mit hydrolytischen Funktionen und katalysieren den Chitinabbau. Es konnte nun gezeigt werden, daß Pflanzen durch die Produktion von Chitinasen eine erhöhte Widerstandsfähigkeit gegen Pathogene erhalten.

Weiterhin ist die Verwendung eines Gens aus Gerstenpflanzen bekannt, dessen Genprodukt für-einen Inhibitor der pilzlichen Proteinsynthese kodiert. Der Einbau eines entsprechenden Inhibitorgens in transgenen Pflanzen führte zu einer verbesserten Pilzresistenz.

Schließlich ist auch bekannt geworden, daß die Verwendung eines Polypeptids aus Aspergillus giganteus aufgrund dessen antifungaler Aktivität Pflanzen vor einem Pilzbefall schützen kann.

Gegenüber diesem Stand der Technik besteht aber das Bedürfnis nach der Schaffung weiterer transgener pathogen-resistenter Organismen. Daneben sind solche Organismen besonders erwünscht, deren Resistenz gegenüber den bekannten Organismen insgesamt vergrößert oder bezüglich der Anzahl der möglichen Pathogene verbreitert wird.

Dieses Problem wird durch eine transgene Pilz-resistente Pflanze mit den Merkmalen des Anspruchs 1 gelöst.

Der Erfindung liegt der überraschende Befund zugrunde, daß der Einbau mindestens zweier unterschiedlicher Gene wie in Anspruch 1 definiert, die für Proteine mit Pilz-inhibierender Wirkung kodieren, in das Genom einer Pflanze dieser zu einer Resistenz gegen Pilze verhilft, die weit über eine additive Wirkung der Gene jeweils für sich hinausgeht.

In den Unteransprüchen werden weitere Ausführungsformen der Erfindung angegeben.

Die Gene kodieren für Genprodukte, die die Vitalität von Pilzen herabsetzen. Insbesondere können die Gene pilzlichen, bakteriellen und pflanzlichen, tierischen oder viralen Ursprungs sein. Die Genprodukte haben die Pilzresistenz fördernde Eigenschaften. Die Genprodukte sind Chitinase (ChiS), Proteinsynthese-Inhibitor (PSI) und antifungales Protein (AFP).

Die transgene Pilz-resistente Pflanze ist eine Pflanze, bei welcher es sich vorzugsweise um Tabak-, Kartoffel-, Erdbeer-Mais-, Raps- oder Tomatenpflanzen handelt.

Beschrieben werden auch DNA-Übertragungsvektoren mit inserierten DNA-Sequenzen, wie sie in dieser Beschreibung im einzelnen angegeben sind.

Gegenstand der Erfindung ist weiterhin ein Verfahren zur Erzeugung Pilz-resistenter Pflanzen, wie sie hier beschrieben werden, wobei in das Genom einer Pflanze mindestens 1 Gen, das für ein Protein mit Pilz-inhibierender Wirkung wie in Anspruch 1 definiert kodiert, transferiert und die Pilz-resistente Pflanze

(a) durch Kreuzung der Pflanze mit einer gegebenenfalls transgenen weiteren Pflanze, die mindestens ein anderes Gen, das für ein anderes Protein mit Pilz-inhibierender Wirkung wie in Anspruch 1 kodiert, enthält, und anschließende Selektion oder
(b) durch Transformation der Pflanze mit mindestens einem anderen Gen, das für ein anderes Protein mit Pilz-inhibierender Wirkung wie in Anspruch 1 definiert kodiert, erhalten wird.

Das Verfahren kann mit DNA-Übertragungsvektoren mit inserierten DNA-Sequenzen entsprechend einem Gen mit Pilz-inhibierender Wirkung, wie hier beschrieben, verwendet werden.

Schließlich ist ein Verfahren zur Erzeugung Pilz-resistenter Pflanzen Gegenstand der Erfindung, wobei zur Transformation in das Genom einer Pflanze Vektoren verwendet werden, die mehr als ein Gen mit Pilz-inhibierender Wirkung umfassen.

Die synergistischen Wirkungen konnten ganz besonders mit transgenen Pilz-resistenten Pflanzen erzielt werden, auf die Gensequenzen transferiert oder transfiziert waren, die für Proteine der anhängenden Sequenzprotokolle A bis C kodierten bzw. diesen entsprachen.

ChiS:

Aus dem Bodenbakterium Serratia marcescens wurde ein 1,8 Kb großes DNA Fragment isoliert, daß für eine Chitinase, genannt. ChiS, kodiert. In vitro Untersuchungen mit gereinigtem ChiS-Protein zeigten, daß es in geringen Konzentrationen bereits das Wachstum von Pilzen wirksam inhibieren kann. Die Ursache für die Inhibition ist, daß das ChiS-Protein über eine Chitinaseaktivität verfügt, mit der die Hyphenspitzen des Pilzes zerstört werden können. Auf diese Weise kann der Pilz nicht weiterwachsen und wird inhibiert.

PSI:

Das PSI-Gen stammt aus Gerste und kodiert für ein Protein, welches die Proteinsynthese von Pilzen inhibiert. Nach in vitro Tests reichen bereits geringe Konzentrationen PSI aus, um diverse Pilze wie zum Beispiel Rhizoctonia solani zu inhibieren.

AFP:

Aus der Fermentationsbrühe von Aspergillus giganteus kann ein Polypeptid isoliert und sequenziert werden, welches über antifungale Aktivität verfügt. Dieses Polypeptid eignet sich als antifungales Agens, z.B. als Sprühmittel und als Konservierungsstoff für technische Produkte und Nahrungs- und Futtermittel. Es kann weiterhin mit anderen pestizid wirksamen Stoffen, Düngemitteln oder Wachstumsregulatoren kombiniert werden. Inhibitorische Aktivitäten gegen Pilze konnten unter Anderem gegen verschiedene Aspergillus-, Fusarien-, Phytophthora- und Trichophyton-Arten nachgewiesen werden.

ChiG und GluG:

Aus bestimmten Gerstearten lassen sich zwei Gene isolieren, die für eine Chitinase (ChiG) bzw. Glukanase (GluG) kodieren. Gereinigtes ChiG-Protein oder GluG-Protein inhibiert in vitro diverse pflanzenpathogene Pilze (u.A. Rhizoctonia solani) (siehe R. Leah et al., Journal of Biological Chemistry, Vol. 266, No. 3 (1991), Seiten 1564-1573).

Die Erfinder haben nun völlig überraschend festgestellt, daß ' die mindestens zweifache kombinierte Expression von PSI, AFP, ChiS, ChiG oder GluG bezüglich der erworbenen Pilzresistenz bei transgenen Pflanzen zu synergistischen Effekten führt. Insbesondere werden die Wirkungen der Einzelsubstanzen in der Kombination deutlich übertroffen. Hierzu gehören die Resistenz gegen den Pilz Rhizoctonia solani, Sclerotinia-Befall, Botrytis-Befall usw.

Erfindungsgemäße Kombinationen sind (DNA und/oder Polypeptide):

(Zweierkombinationen)
ChiS, PSI;
PSI, AFP;

(Dreierkombinationen)
ChiS, GluG, PSI;
GluG, PSI, AFP; PSI, ChiG, AFP;

(Viererkombinationen)
ChiS, GluG, PSI, AFP; ChiS, GluG, PSI, ChiG;

(Fünferkombination)
Chis, GluG, PSI, AFP, ChiG

Gegenstand der Erfindung ist weiterhin die kombinierte Verwendung der Proteine mit Pilz-inhibierender Wirkung, vorzugsweise ChiS, PSI und AFP, gegen Pilze. Kombinierte Verwendung bedeutet hier auch, daß mindestens eine erste Pilz-inhibierende Substanz von der Pflanze exprimiert und mindestens eine zweite Substanz, die Pilz-inhibierende Wirkung hat, von außen auf die Pflanze aufgebracht wird.

Zu den erfindungsgemäßen Mitteln zählen auch jene, die die oben genannten Proteine in mindestens zweifacher Kombination enthalten. Die erfindungsgemäßen Mittel können neben den Proteinen weitere Wirkstoffe enthalten. Diese weiteren Wirkstoffe können Pestizide, Düngemittel und/oder Wachstumsregulatoren sein, die erfindungsgemäßen Mittel können zudem in unterschiedlichen Formulierungen bereitgestellt werden, wie Konzentrate, Emulsionen, Pulver, Formulierungen auf Trägerstoffen, Mischungen mit anderen Wirkstoffen, etc. Besonders bevorzugt wird die Kombination ChiS/PSI und AFP/PSI. Diese Proteine können besonders wirksam zur Wachstumshemmung von Rhizoctonia solani, insbesondere bei Tabakpflanzenkulturen, eingesetzt werden.

Gegenstand der Erfindung ist auch die Verwendung einer DNA-Sequenz in einem erfindungsgemäßen Verfahren, die mindestens für ein Polypeptid der Sequenzen A bis C kodiert, bzw. eine Pilz-resistente Pflanze, wobei deren Genom mindestens zwei unterschiedliche Gene unter der Kontrolle aktiver Promotoren mit Pilz-inhibierender Wirkung enthält, wie sie in Anspruch 1 definiert sind. Die Erfindung beschreibt weiterhin DNA-Sequenzen, die mit einer DNA-Sequenz hybridisieren, welche für Polypeptide der Aminosäuresequenzen A bis C kodiert, wobei diese DNA-Sequenz natürlichen synthetischen oder halbsynthetischen Ursprungs sein kann und mit der zuvor genannten DNA-Sequenz durch Mutationen, Nukleotidsubstitutionen, Nukleotiddeletionen,

Nukleotidinsertionen und Inversionen von Nukleotidfolgen verwandt sein kann und für ein Polypeptid mit Pilz-inhibierender Wirksamkeit kodiert. Beschrieben wird weiter noch ein rekombinantes DNA-Molekül, welches mindestens eine DNA-Sequenz nach den vorstehenden Ausführungen enthält, wobei dieses DNA-Molekül als Klonierungs- oder Expressionsvektor vorliegen kann.

Beschrieben werden auch entsprechende Wirtsorganismen und Zwischenwirte, die mit einem rekombinanten DNA-Molekül nach den vorstehenden Ausführungen transformiert sind. Als Zwischenwirt bei der Erzeugung einer Pilz-resistenten transgenen Pflanze werden Bakterienstämme bevorzugt, insbesondere sogenannte Agrobakterienstämme.

Gegenstand der Erfindung sind weiterhin die nach dem erfindungsgemäßen Verfahren erhaltenen transgenen Pilz-resistenten Pflanzen, insbesondere Tabak-, Kartoffel-, Mais-, Erbsen-, Raps- und Tomatenpflanzen.

Die beschriebenen DNA-Sequenzen werden in der Regel zusammen mit einem Promotor transferiert. Promotorsequenzen werden vom pflanzlichen Transskriptionsapparat erkannt und führen somit zu einer konstitutiven Expression des mit ihnen verbundenen Gens in Pflanzen. Der Promotor kann aber auch Pathogen-induzierbar und/oder verwundungs-induzierbar (WUN1) und/oder gewebespezifisch und/oder entwicklungsspezifisch sein.

Die zur Durchführung der Erfindung erforderlichen gentechnologischen Arbeiten, insbesondere zur Expression des Gens in Pflanzen, sind allgemein bekannt. Beispielsweise aus der Veröffentlichung von Maniatis et al. in "Molecular cloning: A laboratory manual", Cold Spring Harbour (1982)

Die Erfindung wird in den folgenden Beispielen näher erläutert.

Alle mokekularbiologischen Standard-Methoden wurden, sofern nicht anders angegeben, wie bei Maniatis et al. "Molecular cloning: a laboratory manual", Cold Spring Harbour, (1982) beschrieben, durchgeführt.

Die für die Aminosäuresequenzen A bis C kodierende DNA wurde zunächst in an sich bekannter Weise kloniert und dann durch Konjugation nach A. Tumefaciens LBA 4404 (A. Hoekema et al., Nature 303, 179-180) transferiert. Dies geschah nach der von Van Haute et al. in EMBO J. 2, 411-418 (1983), beschriebenen Methode.

Die Überprüfung der DNA-Transfers in das Agrobakterium erfolgte durch die Isolierung von Agrobakterien-DNA nach der von Ebert et al. in Proc. Natl. Acad. Sci. USA 84 5745-5749 (1987), geschilderten Methode. Die Restriktionsspaltung der DNA, der Transfer auf Hybond-N-Membran (Amersham) und die Hybridisierung gegen eine radioaktiv markierte DNA-Sonde gaben Aufschluß über einen erfolgreichen DNA-Transfer in das Agrobakterium.

Mittels des transformierten Agrobakteriums wurden wiederum Tabak-, Raps-, Erdbeer-, Tomaten- und Kartoffelpflanzen transformiert.

Die zur Infektion benötigten Agrobakterien LBA 4404 wurden in selektivem Antibiotika-Medium angezogen (P. Zambrisky et al. in EMBO J., 1, 147-152 (1983)), durch Zentrifugation sedimentiert und in YEB-Medium ohne Antibiotika gewaschen (YEB = 0,5% Fleisch-Extrakt; 0,2% Hefeextrakt; 0,5% Pepton; 0,5% Saccharose; 2mM $MgSO_4$). Nach erneuter Sedimentation und Aufnahme in $MgSO_4$ konnten die Bakterien zur Infektion verwendet werden.

Zur Infektion wurde die sogenannte Blattscheibenmethode eingesetzt.

Für die Blattscheiben-Infektion wurden sterile Blätter verwendet. Etwa 1 cm große Blattstücke werden in die zuvor beschriebene Agrobakteriensuspension eingetaucht und anschließend auf 3MS-Medium überführt (Medium nach T. Murashige und F. Skoog in Physiol. Plant., 15, 473-497 (1962); 3MS = MS + 3% Saccharose). Nach zweitägiger Inkubation bei 16 Stunden Licht und 25°C bis 27°C wurden die Blattstücke auf MSC16-Medium (nach T. Murashige (siehe oben); MSC16 = MS + 0,5 µg/ml BAP + 0,1 µg/ml NAA + 100 µg/ml Kanamycinsulfat + 500 µg/ml Claforan) überführt. Nach 4-6 Wochen erscheinende Sprosse wurden abgeschnitten und auf MSC15-Medium (nach Murashige (siehe oben); MSC15 = MS + 2% Saccharose, 500 µg/ml Claforan + 100 µg/ml Kanamycinsulfat) umgesetzt. Sprosse mit Wurzelbildung wurden weiter analysiert.

Monokotyledone Pflanzen (u. a. Mais), zum Teil aber auch dikolyte Pflanzen wurden mittels direktem Gentransfer in Protoplasten transformiert. Diese Protoplasten wurden anschließend zu intakten Pflanzen regeneriert (Beispiel: J. Potrykus in Biotechnology 8 (1990) 535).

Die erhaltenen transgenen Pflanzen wurden zu Testzwecken mit dem Pilz Rhizoctonia solani infiziert. Hierzu wurden Pilzkulturen gezüchtet und in Einheitserde gründlich vermischt. Diese Erde wurde dann in einer Schale verteilt und mit den zu testenden Pflanzen bepflanzt.

Zur Auswertung wurde jeder Pflanze einer Schale ein Wert von 0 bis 3 zugeordnet. Daraus konnte für jede Pflanzenlinie ein Index berechnet werden, der sich aus der Summe der Werte ergab. Die Einteilung ist wie folgt:

0 = Ohne Symptome (gesund)
1 = leicht reduzierte Größe (gegenüber einer nicht-infizierten Kontrolle); kein bis sehr geringer sichtbarer Befall
2 = starke Wachstumsreduktion; schwere Befallssymptome

3 = tot

Die Bewertung erfolgt jeweils 14 Tage nach Start der Versuchsreihe.

Beispiel 1:

Pilzinhibitionstest mit kombinierten Proteinen

Es sollte zunächst einmal gezeigt werden, daß die hier verwendeten Proteine in ihrer Kombination synergistische Wirkungen haben. Hierzu wurden in vitro Pilzwachstumstests durchgeführt.

Hierbei wurde eine definierte Menge an Rhizoctonia solani Pilzmycel mit 100 µl-Kartoffel-Dextroselösung versetzt und in Mikrotiterplatten bei 25°C inkubiert. Dabei korreliert das Wachstum des Pilzes mit der Zunahme der optischen Dichte bei 405 Nanometer linear. Die inhibierende Wirkung von Proteinen kann anhand eines geringeren Anstiegs der optischen Dichte nachgewiesen werden.

Aus einer Flüssigkultur von R. Solani wurden 2-3 Mycelbällchen entnommen, in einem Eppendorfgefäß mit 100 µl KGB-Medium versetzt und mit einem Glasmörser vorsichtig homogenisiert. Diese Suspension wurde dann mit 10 ml KGB-Medium gemischt und durch ein steriles 100 µm Sieb gegeben. Die optische Dichte dieser Mycelfragment-Suspension (100 µl-Aliquot) wurde durch Zugabe von Medium auf einen Wert von 0,06-0,07 bei 405 Nanometer eingestellt. Je 100 µl wurden auf eine Mikrotiterplatte gegeben und mit den zu testenden Proteinen versetzt. Pro Ansatz wurden 7 Parallelen gemessen. Als Kontrolle dienen Ansätze, die mit den entsprechenden Mengen an Puffer versetzt wurden. Die Platten wurden über 48 Stunden bei 25°C im Dunkeln inkubiert und die optische Dichte der Kulturen in regelmäßigen Abständen gemessen.

Ob zwei Proteine bei der Wachstumshemmung des Pilzes in additiver synergistischer oder antagonistischer Weise zusammenwirken, läßt sich aus den gemessenen Daten mit Hilfe der im folgenden beschriebenen und allgemeinen angewandten Colby-Formel errechnen (S. R. Colby in Wheeds, 15 (1967), 20-22).

Hierzu war es zunächst notwendig, die bei einem additiven Verhalten theoretisch zu erwartende Wachstumshemmung E (der erwartete Wirkungsgrad) zu berechnen. Dieser ist gegeben durch:

$$E = W1 + W2 - ((W1 \times W2)/100)$$

Dabei geben W1 und W2 die Wirkungsgrade der einzelnen Proteine an, worunter man die prozentuale Abweichung der Wachstumskurve (in Anwesenheit des Proteins) von der unbehandelten Kontrolle versteht. Der Wirkungsgrad für ein Protein ist (zu einem bestimmten Zeitpunkt der Wachstumskurve) gegeben durch:

$$W1 = (OD(K) - OD(P))/OD(K) \times 100 \qquad \text{(Prozent)}$$

Hierbei ist OD(K) die optische Dichte der unbehandelten Kontrolle und OD(P) die optische Dichte der mit dem Protein behandelten Kultur.

Bei der kombinierten Anwendung von zwei Proteinen waren somit folgende Aussagen möglich: Ist der im Experiment gemessene Wirkungsgrad G gleich dem Erwartungswert E, so handelt es sich um ein additives Verhalten. Ist G hingegen größer als E, so liegt synergistisches Verhalten vor.

Unter Verwendung dieses Prüfmodells ergaben sich für die im Beispiel verwendeten Proteine ChiS, PSI, AFP, ChiG und GluG überraschenderweise synergistische Hemmeffekte gegen diverse Pilze, wobei diese Effekte sowohl durch die Kombination zweier Proteinarten, als auch durch die Mehrfachkombination der obengenannten Proteine erreicht wurde.

Beispielsweise wurde aus der Kombination von ChiS und PSI-Protein, bzw. aus der Kombination von AFP- und PSI-Protein gegen den Pilz Rhizoctonia solani folgende Werte ermittelt (je zwei verschiedene ChiS und AFP-Konzentrationen bei konstanter RIP-Konzentration):

ChiS + PSI:

| | |
|---|---|
| Die Erwartungswerte waren: | E1 = 29,9% und E2 = 44,5% |
| Die gemessenen Werte waren: | G1 = 60,4% und G2 = 64,1% |

Die Proteine ChiS und PSI wirken also bei der Wachstumshemmung von R. Solani in synergistischer Weise zusammen.

Die Fig. 1 zeigt die Ergebnisse, die mit der Kombination der Proteine, als auch mit den Einzelsubstanzen erhalten wurden. Nach der Figur werden verschiedene ChiS-Konzentrationen (0,5 µg/ml bzw. 0,05 µg/ml) mit PSi-Protein (1,0 µg/ml) kombiniert.

AFP + PSI:

| | |
|---|---|
| Die Erwartungswerte waren: | E1 = 39,9% und E2 = 41,9% |
| Die gemessenen Werte waren: | G1 = 57,7% und G2 = 65,4% |

Auch die Kombination AFP und PSI zeigt demnach eine synergistische Wachstumshemmung des Pilzes R. Solani an. In der Fig. 2 werden die Testergebnisse bei verschiedenen AFP-Konzentrationen (0,4 µg/ml bzw. 0,04 µg/ml) mit PSI-Protein (1,0 µg/ml) kombiniert angegeben.

Beispiel 2:

Transgene Pflanzen

Um die erfindungsgemäßen Organismen mit synergistisch zusammenwirkenden DNA-Sequenzen zu erhalten, wurden zunächst transgene Pflanzen erzeugt, die mindestens eines der synergistisch zusammenwirkenden Gene enthielten.

ChiS in transgenen Pflanzen

Es wurde zunächst ein ChiS-Gen mit pflanzlichen Regulations-sequenzen fusioniert.

Ein 1,8 Kb großes ChiS-Gen wurde durch die Verwendung von synthetischen Oligonukleotiden nach der Dideoxy-sequenzierungsmethode von Sanger et al. in Proc. Natl. Acad. Sci. USA, 74, (1977), 5463-5467, sequenziert.

Der aus dem Blumenkohlmosaikvirus (CamV) stammende 35S-Promotor (400 bp (nach Töpfer et al. in Nucl. Acid. Res., 15 (1987) 5890)) wurde transskriptionell mit dem ChiS-Gen fusioniert. 3' vom ChiS-Gen wurde das 0,2 Kb große Terminationssignal des 35S-Gens des CamV verwendet, dessen Funktionalität in dikotylen Pflanzen bekannt ist. Das chimäre Gen 35S-ChiS wurde in den Vektor pLS034 kloniert, mittels des Agrobakteriums tumefaciens-Transformationssystems in Tabak- und Kartoffelpflanzen transformiert und Kanamycin-resistente Pflanzen regeneriert.

In den erhaltenen Pflanzen konnte sowohl das ChiS-Gen als auch die entsprechende mRNA sowie das Genproduktprotein nachgewiesen werden.

PSI in transgenen Pflanzen

Aus reifen Gerstesamen (Hordeum vulgare L. cv. Piggy) wurde zunächst PolyA$^{+-}$ RNA isoliert und in einer cDNA-Genbank in λ-gt-11-Phagen abgelegt. Die Einzelheiten des Verfahrens sind R. Lea in Plant. Biol., 12 (1989), 673-682, zu entnehmen. Mit Hilfe monospezifischer PSI-Antikörper wurden dann cDNA-Klone identifiziert.

Im Anschluß daran wurden die PSI-positiven λ-gt-11-Phagen isoliert, weiter kloniert und nach der oben angegebenen Dideoxy-sequenzierungsmethode von Sanger et al. sequenziert. Die in E. Coli geklonte DNA wurde dann in der oben beschriebenen Weise durch Konjugation auf das Agrobakterium tumefaciens LBA4404 übertragen.

Sowohl das transferierte Gen als auch mRNA und Genprodukt konnten in entsprechenden transgenen Tabak-, Kartoffel-, Raps-, Erdbeer- und Tomatenpflanzen nachgewiesen werden.

AFP in transgenen Pflanzen

Die cDNA-Sequenz des antifungischen Peptids wird für die Klonierung im Vektor mit Enden versehen, die in BamH1- und SalI-Restriktionsschnittstellen ligiert werden können. Als Klonierungsvektor wurde pDH51 (Pietrzak et al. in Nucl. Acids Res. 14 (1986), 5857) verwendet. Der Vektor pDH51 wurde mit den Restriktionsenzymen BamH1 und Sal1 zwischen Promotor und Terminator geöffnet. Der Vektor pDH51 ist ein pUC18-Derivat, das Promotor- und Terminatorsequenzen des 35S-Transkripts aus Blumenkohlmosaikvirus enthält. Diese Sequenzen werden vom pflanzlichen Transskriptionsapparat erkannt und führen zu einer starken konstitutiven Expression des mit ihnen verbundenen Gens in Plfanzen. Die DNA des antifungischen Peptids wird dann über die BamH1 und SalI-Schnittstelle in den Vektor kloniert. Schließlich wird die Transskriptionseinheit - Promotor, Gen und Terminator - mit dem Restriktionsenzym EcoRI aus dem Vektor herausgeschnitten und in einen Pflanzentransformationsvektor kloniert. Als Pflanzentransformationsvektor können zum Beispiel folgende Vektoren bzw. ihre Derivate verwendet werden:

pOCA18 (Olszewski et al. in Nucl. Acids Res., 16 (1988), 10765) pPCV310 (Koncz und Shell in MGG 204 (1986), 383) und pBin19 (Bevan et al. Nucl. Acids. Res. 12 (1984) 8711)

Nachdem die Transskriptionseinheit und der Vektor über die EcoRI-Schnittstelle ligiert wurde, wurde das Konstrukt in den Agrobakterienstamm MP90RK (Koncz und Shell (siehe oben)) oder IHA101 (Hood et al. in J. Bacteriol. 168 (1986), 1291) konjugiert.

Transgene Tabak-, Kartoffel-, Erdbeer-, Raps- und Tomatenpflanzen wurden dann nach der oben beschriebenen Methode transformiert. Transformierte Sprosse werden aufgrund der mitübertragenen Resistenz gegen das Antibiotikum Kanamycin selektioniert. Durch DNA-Analyse (Southern Blotting), RNA-Analyse (Northern Blotting) und Proteinanalyse mit spezifischen Antikörpern (Western Blotting) wurde die Expression des antifungischen Proteins in den transformierten Nutzpflanzen überprüft und bestätigt.

ChiG und GluG in transgenen Pflanzen

Analog zu den zuvor beschriebenen Pflanzen konnten ChiG- bzw. GluG-transgene Pflanzen erhalten werden, die sowohl Southern-, Northern- als auch Western-positiv waren.

ChiS, PSI, AFP, ChiG, GluG in transgenen monokotylen Pflanzen

Die zuvor genannten Gene konnten mittels direktem Gentransfer in das Genom monokotyler Pflanzen wie beispielsweise Mais integriert werden. Hierbei wurden transgene Pflanzen erhalten, die sowohl Southern- als auch Northern- und Western-positiv waren.

Kombination verschiedener Pilzresistenzgene in transgenen Pflanzen

Die zuvor erhaltenen Tabak-, Mais-, Raps-, Erdbeer-, Kartoffelund Tomatenpflanzen wurden miteinander gekreuzt und auf Pflanzen selektioniert, die jeweils die Pilzresistenzgene beider Eltern beeinhalteten. Darüberhinaus wurden transgene Pflanzen dadurch erhalten, daß sie zunächst mit einem und dann mit einem oder mehreren weiteren Gen transformiert wurden. Schließlich wurden auch noch Pflanzen mit Vektoren transformiert, die verschiedene Resistenzgene beeinhalteten. Mit diesem Pflanzengut wurden Pilzresistenztests gemacht. Überraschenderweise sind in allen Fällen nicht nur additive Effekte bezüglich der Pilzresistenz zu beobachten, sondern synergistische Effekte.

Beispielsweise zeigt eine Tabakpflanze, die ChiS und PSI exprimiert, eine wesentlich stärkere Widerstandsfähigkeit gegen Rhizoctonia-Befall als die Pflanzen, die entweder nur ChiS oder PSI exprimierten, oder die sich aus der additiven Widerstandsfähigkeit ergeben würde.

Ein synergistischer Hemmeffekt ergibt sich auch aus der kombinierten Expression von PSI- und AFP-transgenen Tabak gegen Rhizoctonia solani Befall. Auch die zwei- oder mehrfache Kombination verschiedener Gene (ChiS, RIP, AFP, ChiG und GluG) in den unterschiedlichsten transgenen Pflanzen führte zu synergistischen Hemmeffekten gegen diverse Pilze.

Während Wildtyppflanzen bei Tests mit 20 Sämlingen Indexwerte von 38 bis 46 aufweisen, erweist sich bei erfindungsgemäßen transgenem Tabak, daß dieser in Anwesenheit des Pilzes Rhizoctonia solani so gut wächst wie Kontrollpflanzen (Indexwert 10-12), die auf Rhizoctonia-freiem Boden kultiviert wurden.

Sequenzprotokolle 1 bis 3 (AFP):

Seq IDNo.: 1 bis 3 (1)
Art der Sequenz: Vollständige Nukleotidsequenz mit entsprechendem Protein, soweit es durch ein offenes Ableseraster codiert wird, wirksames Protein (3)
Sequenzlänge: 51 Aminosäuren (3)
Strangform: Einzelstrang
Topologie: linear
Art des Moleküls: cDNA
Ursprüngliche Herkunft: Fermentationsbrühe von Aspergillus giganteus
Name: Antifungisches Peptid (AFP)
Merkmale (1) :
Offenes Ableseraster von 177 Nukleotiden, die N-terminale

Aminosäure des wirksamen Proteins ist mit * markiert.

Eigenschaften: Antifungisches Agens, insbesondere gegen Rhizoctonia solani, verschiedene Aspergillus-, Fusarien- und Trichophyton-Arten.

SEQ ID NO: 1:

```
TTGCCACCCC CGTTGAAGCC GATTCTCTCA CCGCTGGTGG TCTGG ATG CAA GAG        54
                                                  Met Gln Glu
                                                   1

ATG AGA GCG CGG GTT TTG GCC ACA TAC AAT GGC AAA TGC TAC AAG AAG     102
Met Arg Ala Arg Val Leu Ala Thr Tyr Asn Gly Lys Cys Tyr Lys Lys
     5                  10                  15

GAT AAT ATC TGC AAG TAC AAG GCA CAG AGC GGC AAG ACT GCC ATT TGC     150
Asp Asn Ile Cys Lys Tyr Lys Ala Gln Ser Gly Lys Thr Ala Ile Cys
 20                  25                  30                  35

AAG TGC TAT GTC AAA AAG TGC CCC CGC GAC GGC GCG AAA TGC GAG TTT     198
Lys Cys Tyr Val Lys Lys Cys Pro Arg Asp Gly Ala Lys Cys Glu Phe
                 40                  45                  50

GAC AGC TAC AAG GGG AAG TGC TAC TGC TAGACGGTGA GCGAAGGGAC           245
Asp Ser Tyr Lys Gly Lys Cys Tyr Cys
             55                  60

GAAGTAGGCT GGGGGTTATT TTACTCTGCT                                    275
```

SEQ ID NO: 2 (Aminosäuresequenz der SEQ ID NO: 1)

```
Met Gln Glu Met Arg Ala Arg Val Leu Ala Thr Tyr Asn Gly Lys Cys
 1               5                  10                  15

Tyr Lys Lys Asp Asn Ile Cys Lys Tyr Lys Ala Gln Ser Gly Lys Thr
             20                  25                  30

Ala Ile Cys Lys Cys Tyr Val Lys Lys Cys Pro Arg Asp Gly Ala Lys
         35                  40                  45

Cys Glu Phe Asp Ser Tyr Lys Gly Lys Cys Tyr Cys
     50                  55                  60
```

SEQ ID NO: 3:

```
Ala Thr Tyr Asn Gly Lys Cys Tyr Lys Lys Asp Asn Ile Cys Lys Tyr
1               5                   10                  15

Lys Ala Gln Ser Gly Lys Thr Ala Ile Cys Lys Cys Tyr Val Lys Lys
            20                  25                  30

Cys Pro Arg Asp Gly Ala Lys Cys Glu Phe Asp Ser Tyr Lys Gly Lys
        35                  40                  45

Cys Tyr Cys
    50
```

Sequenzprotokolle 4 bis 7 (PSI) :

Seq IDNo.: 4 bis 7
Art der Sequenz: Nukleotid mit entsprechendem Protein Sequenzlänge: 1078 Basenpaare (6 und 7 = unvollständiger PSI-cDNA-Klon)
Strangform: Einzelstrang
Topologie: linear
Art des Moleküls: komplementär DNA
Urspüngliche Herkunft: Gerstesamen (Hordeum vulgare L.cv. Piggy)
Unmittelbare experimentelle Herkunft: cDNA-Genbank in 2-gt-11-Phagen
Name: Proteinsyntheseinhibitor
Merkmale:
42 bp-lange 5'-nicht-übersetzende Region
Offenes Ableseraster von 843 Basenpaaren (das Stopcodon ist mit. einem Sternchen markiert)
193 basenpaarlanges 3'-nicht-übersetztes Ende,
mögliche Polyadenylierungssignale sind unterstrichen Eigenschaften:
Antifungal wirksam, insbesondere gegen Sporen von Trichoderma reesii und fusarium sporotrichoides sowie gegen Rhizoctonia solani.

SEQ ID NO: 4:

```
CTTAATAGCA CATCTTGTCC GTCTTAGCTT TGCATTACAT CC ATG GCG GCA AAG        54
                                               Met Ala Ala Lys
                                                -1   1

ATG GCG AAG AAC GTG GAC AAG CCG CTC TTC ACC GCG ACG TTC AAC GTC      102
Met Ala Lys Asn Val Asp Lys Pro Leu Phe Thr Ala Thr Phe Asn Val
     5                  10                  15

CAG GCC AGC TCC GCC GAC TAC GCC ACC TTC ATC GCC GGC ATC CGC AAC      150
Gln Ala Ser Ser Ala Asp Tyr Ala Thr Phe Ile Ala Gly Ile Arg Asn
 20                  25                  30                  35

AAG CTC CGC AAC CCG GCG CAC TTC TCC CAC AAC CGC CCC GTG CTG CCG      198
Lys Leu Arg Asn Pro Ala His Phe Ser His Asn Arg Pro Val Leu Pro
                 40                  45                  50

CCG GTC GAG CCC AAC GTC CCG CCG AGC AGG TGG TTC CAC GTC GTG CTC      246
Pro Val Glu Pro Asn Val Pro Pro Ser Arg Trp Phe His Val Val Leu
             55                  60                  65

AAG GCC TCG CCG ACC AGC GCC GGG CTC ACG CTG GCC ATT CGG GCG GAC      294
Lys Ala Ser Pro Thr Ser Ala Gly Leu Thr Leu Ala Ile Arg Ala Asp
         70                  75                  80

AAC ATC TAC CTG GAG GGC TTC AAG AGC AGC GAC GGC ACC TGG TGG GAG      342
Asn Ile Tyr Leu Glu Gly Phe Lys Ser Ser Asp Gly Thr Trp Trp Glu
     85                  90                  95

CTC ACC CCG GGC CTC ATC CCC GGC GCC ACC TAC GTC GGG TTC GGC GGC      390
Leu Thr Pro Gly Leu Ile Pro Gly Ala Thr Tyr Val Gly Phe Gly Gly
100                 105                 110                 115

ACC TAC CGC GAC CTC CTC GGC GAC ACC GAC AAG CTG ACC AAC GTC GCT      438
Thr Tyr Arg Asp Leu Leu Gly Asp Thr Asp Lys Leu Thr Asn Val Ala
                120                 125                 130

CTC GGC CGG CAG CAG CTG GCG GAC GCG GTG ACC GCC CTC CAC GGG CGC      486
Leu Gly Arg Gln Gln Leu Ala Asp Ala Val Thr Ala Leu His Gly Arg
            135                 140                 145

ACC AAG GCC GAC AAG CCG TCC GGC CCG AAG CAG CAG CAG GCG AGG GAG      534
Thr Lys Ala Asp Lys Pro Ser Gly Pro Lys Gln Gln Gln Ala Arg Glu
        150                 155                 160

GCG GTG ACG ACG CTG CTC CTC ATG GTG AAC GAG GCC ACG CGG TTC CAG      582
Ala Val Thr Thr Leu Leu Leu Met Val Asn Glu Ala Thr Arg Phe Gln
    165                 170                 175

ACG GTG TCT GGG TTC GTG GCC GGG TTG CTG CAC CCC AAG GCG GTG GAG      630
Thr Val Ser Gly Phe Val Ala Gly Leu Leu His Pro Lys Ala Val Glu
180                 185                 190                 195

AAG AAG AGC GGG AAG ATC GGC AAT GAG ATG AAG GCC CAG GTG AAC GGG      678
Lys Lys Ser Gly Lys Ile Gly Asn Glu Met Lys Ala Gln Val Asn Gly
                200                 205                 210
```

```
TGG CAG GAC CTG TCC GCG GCG CTG CTG AAG ACG GAC GTG AAG CCT CCG        726
Trp Gln Asp Leu Ser Ala Ala Leu Leu Lys Thr Asp Val Lys Pro Pro
            215                 220                 225

CCG GGA AAG TCG CCA GCG AAG TTC GCG CCG ATC GAG AAG ATG GGC GTG        774
Pro Gly Lys Ser Pro Ala Lys Phe Ala Pro Ile Glu Lys Met Gly Val
        230                 235                 240

AGG ACG GCT GTA CAG GCC GCC AAC ACG CTG GGG ATC CTG CTG TTC GTG        822
Arg Thr Ala Val Gln Ala Ala Asn Thr Leu Gly Ile Leu Leu Phe Val
    245                 250                 255

GAG GTG CCG GGT GGG TTG ACG GTG GCC AAG GCG CTG GAG CTG TTC CAT        870
Glu Val Pro Gly Gly Leu Thr Val Ala Lys Ala Leu Glu Leu Phe His
260                 265                 270                 275

GCG AGT GGT GGG AAA TAGGTAGTTT TCCAGGTATA CCTGCATGGG TAGTGTAAAA        925
Ala Ser Gly Gly Lys
                280

GTCGAATAAA CATGTCACAG AGTGACGGAC TGATATAAAT AAATAAATAA ACGTGTCACA      985

GAGTTACATA TAAACAAATA AATAAATAAT TAAAAATGTC CAGTTTA                   1032
```

SEQ ID NO: 5 (Aminosäuresequenz der SEQ ID NO: 4)

```
Met Ala Ala Lys Met Ala Lys Asn Val Asp Lys Pro Leu Phe Thr Ala
 -1   1               5                  10                  15

Thr Phe Asn Val Gln Ala Ser Ser Ala Asp Tyr Ala Thr Phe Ile Ala
                 20                  25                  30

Gly Ile Arg Asn Lys Leu Arg Asn Pro Ala His Phe Ser His Asn Arg
             35                  40                  45

Pro Val Leu Pro Pro Val Glu Pro Asn Val Pro Pro Ser Arg Trp Phe
         50                  55                  60

His Val Val Leu Lys Ala Ser Pro Thr Ser Ala Gly Leu Thr Leu Ala
     65                  70                  75

Ile Arg Ala Asp Asn Ile Tyr Leu Glu Gly Phe Lys Ser Ser Asp Gly
 80                  85                  90                  95

Thr Trp Trp Glu Leu Thr Pro Gly Leu Ile Pro Gly Ala Thr Tyr Val
                100                 105                 110

Gly Phe Gly Gly Thr Tyr Arg Asp Leu Leu Gly Asp Thr Asp Lys Leu
            115                 120                 125

Thr Asn Val Ala Leu Gly Arg Gln Gln Leu Ala Asp Ala Val Thr Ala
        130                 135                 140
```

Leu His Gly Arg Thr Lys Ala Asp Lys Pro Ser Gly Pro Lys Gln Gln
145 150 155

Gln Ala Arg Glu Ala Val Thr Thr Leu Leu Leu Met Val Asn Glu Ala
160 165 170 175

Thr Arg Phe Gln Thr Val Ser Gly Phe Val Ala Gly Leu Leu His Pro
180 185 190

Lys Ala Val Glu Lys Lys Ser Gly Lys Ile Gly Asn Glu Met Lys Ala
195 200 205

Gln Val Asn Gly Trp Gln Asp Leu Ser Ala Ala Leu Leu Lys Thr Asp
210 215 220

Val Lys Pro Pro Pro Gly Lys Ser Pro Ala Lys Phe Ala Pro Ile Glu
225 230 235

Lys Met Gly Val Arg Thr Ala Val Gln Ala Ala Asn Thr Leu Gly Ile
240 245 250 255

Leu Leu Phe Val Glu Val Pro Gly Gly Leu Thr Val Ala Lys Ala Leu
260 265 270

Glu Leu Phe His Ala Ser Gly Gly Lys
275 280

SEQ ID NO: 6:

```
GCG GTG ACG ACG CTG CTC CTC ATG GTG AAC GAG GCC ACG CGG TTC CAG        48
Ala Val Thr Thr Leu Leu Leu Met Val Asn Glu Ala Thr Arg Phe Gln
  1               5                  10                  15

ACG GTG TCG GGG TTC GTG GCC GGG CTG CTG CAC CCC AAG GCG GTG GAG        96
Thr Val Ser Gly Phe Val Ala Gly Leu Leu His Pro Lys Ala Val Glu
             20                  25                  30

AAG AAG AGC GGG AAG ATC GGC AAT GAG ATG AAG GCC CAG GTG AAC GGG       144
Lys Lys Ser Gly Lys Ile Gly Asn Glu Met Lys Ala Gln Val Asn Gly
         35                  40                  45

TGG CAG GAC CTG TCC GCG GCG CTG CTG AAG ACG GAC GTG AAG CCC CCG       192
Trp Gln Asp Leu Ser Ala Ala Leu Leu Lys Thr Asp Val Lys Pro Pro
     50                  55                  60

CCG GGA AAG TCG CCA GCG AAG TTC ACG CCG ATC GAG AAG ATG GGC GTG       240
Pro Gly Lys Ser Pro Ala Lys Phe Thr Pro Ile Glu Lys Met Gly Val
 65                  70                  75                  80

AGG ACT GCT GAG CAG GCT GCG GCT ACT TTG GGG ATC CTG CTG TTC GTT       288
Arg Thr Ala Glu Gln Ala Ala Ala Thr Leu Gly Ile Leu Leu Phe Val
                 85                  90                  95

GAG GTG CCG GGT GGG TTG ACG GTG GCC AAG GCG CTG GAG CTG TTT CAT       336
Glu Val Pro Gly Gly Leu Thr Val Ala Lys Ala Leu Glu Leu Phe His
            100                 105                 110

GCG AGT GGT GGG AAA TAGGTAGTTT TGCAGGTATA CCTGCATGGG TAAATGTAAA       391
Ala Ser Gly Gly Lys
        115

AGTCGAATAA AAATGTCACA GAGTGACGGA CTGATATAAA TAAATTAATA AACATGTCAT     451

CATGAGTGAC AGACTGATAT AAATAAATA                                       480
```

SEQ ID NO: 7 (Aminosäuresequenz der SEQ ID NO: 6)

```
Ala Val Thr Thr Leu Leu Leu Met Val Asn Glu Ala Thr Arg Phe Gln
 1               5                  10                  15

Thr Val Ser Gly Phe Val Ala Gly Leu Leu His Pro Lys Ala Val Glu
            20                  25                  30

Lys Lys Ser Gly Lys Ile Gly Asn Glu Met Lys Ala Gln Val Asn Gly
        35                  40                  45

Trp Gln Asp Leu Ser Ala Ala Leu Leu Lys Thr Asp Val Lys Pro Pro
    50                  55                  60

Pro Gly Lys Ser Pro Ala Lys Phe Thr Pro Ile Glu Lys Met Gly Val
65                  70                  75                  80

Arg Thr Ala Glu Gln Ala Ala Ala Thr Leu Gly Ile Leu Leu Phe Val
                85                  90                  95

Glu Val Pro Gly Gly Leu Thr Val Ala Lys Ala Leu Glu Leu Phe His
            100                 105                 110

Ala Ser Gly Gly Lys
        115
```

Sequenzprotokoll 8 (Chis):

Seq IDNo.: 8
Art der Sequenz: Nukleotid
Strangform: Einzelstrang (der aktivierte Strang ist Doppelstrang)
Topologie: linear
Art des Moleküls: cDNA
Unmittelbare experimentelle Herkunft: Plasmid pLChiS aus dem E. Coli-Stamm A 5187
Ursprüngliche Herkunft: Cosmidbank aus Serratia Marcescens
Name: Chis-Protein (Chitinase)
Eigenschaften: Exo-Chitinase

SEQ ID NO: 8:

CAGGGCGTTG TCAATAATGA CAACACCCTG GCTGAAGAGT GTGGTGCAAT ACTGATAAAT 60
ATTTATCTTT CCTTAATAGA AAATTCACTA TCCTTATTTG TCATGTTTTC TTTTATTTAT 120
ATGAAAATAA ATTCACGCTT GCTGAATAAA ACCCAGTTGA TAGCGCTCTT GTTTTTGCGC 180
CTTTTTTATT TATAGTACTG AATGTACGCG GTGGGAATGA TTATTTCGCC ACGTGGAAAG 240
ACGCTGTTGT TATTTATTGA TTTTAACCTT CGCGGATTAT TGCGGAATTT TTTCGCTTCG 300
GCAATGCATC GCGACGATTA ACTCTTTTAT GTTTATCCTC TCGGAATAAA GGAATCAGTT 360
ATGCGCAAAT TTAATAAACC GCTGTTGGCG CTGTTGATCG GCAGCACGCT GTGTTCCGCG 420
GCGCAGGCCG CCGCGCCGGG CAAGCCGACC ATCGCCTGGG GCAACACCAA GTTCGCCATC 480
GTTGAAGTTG ACCAGGCGGC TACCGCTTAT AATAATTTGG TGAAGGTAAA AAATGCCGCC 540
GATGTTTCCG TCTCCTGGAA TTTATGGAAT GGCGACACCG GCACGACGGC AAAAGTTTTA 600
TTAAATGGCA AAGAGGCGTG GAGTGGTCCT TCAACCGGAT CTTCCGGTAC GGCGAATTTT 660
AAAGTGAATA AAGGCGGCCG TTATCAAATG CAGGTGGCAC TGTGCAATGC CGACGGCTGC 720
ACCGCCAGTG ACGCCACCGA AATTGTGGTA GCCGACACCG ACGGCAGCCA TTTGGCGCCG 780
TTGAAAGAGC CGCTGCTGGA AAAGAATAAA CCGTATAAAC AGAACTCCGG CAAAGTGGTC 840
GGTTCTTATT TCGTCGAGTG GGGCGTTTAC GGGCGCAATT TCACCGTCGA CAAGATCCCG 900
GCGCAAAACC TGACCCACCT GCTGTACGGC TTTATCCCGA TCTGCGGCGG CAATGGCATC 960
AACGACAGCC TGAAAGAGAT TGAAGGCAGC TTCCAGGCGT TGCAGCGCTC CTGCCAGGGC 1020
CGCGAGGACT TCAAAGTCTC GATCCACGAT CCGTTCGCCC CGCTGCAAAA AGCGCAGAAG 1080
GGCGTGACCG CCTGGGATGA CCCCTACAAG GGCAACTTCG GCCAGCTGAT GGCGCTGAAG 1140

```
CAGGCGCATC CTGACCTGAA AATCCTGCCG TCGATCGGCG GCTGGACGCT GTCCGACCCG    1200
TTCTTCTTCA TGGGCGACAA GGTGAAGCGC GATCGCTTCG TCGGTTCGGT GAAAGAGTTC    1260
CTGCAGACCT GGAAGTTCTT CGACGGCGTG GATATCGACT GGGAGTTCCC GGGCGGCAAA    1320
GGCGCCAACC CTAACCTGGG CAGCCCGCAA GACGGGGAAA CCTATGTGCT GCTGATGAAG    1380
GAGCTGCGGG CGATGCTGGA TCAGCTGTCG GTGGAAACCG GCCGCAAGTA TGAGCTGACC    1440
TCCGCCATCA GCGCCGGTAA GGACAAGATC GACAAGGTGG CTTACAACGT TGCGCAGAAC    1500
TCGATGGATC ACATCTTCCT GATGAGCTAC GACTTCTATG GCGCCTTCGA TCTGAAGAAC    1560

CTGGGGCATC AGACCGCGCT GAATGCGCCG GCCTGGAAAC CGGACACCGC CTACACCACG    1620
GTGAACGGCG TCAATGCGCT GCTGGCGCAG GGCGTCAAGC CGGGCAAAAT CGTCGTCGGC    1680
ACCGCCATGT ATGGCCGCGG CTGGACCGGG GTGAACGGCT ACCAGAACAA TATTCCGTTC    1740
ACCGGCACCG CCACCGGGCC GGTTAAAGGC ACCTGGGAGA ACGGTATCGT GGACTACCGC    1800
CAAATCGCCG GCCAGTTCAT GAGCGGCGAG TGGCAGTATA CCTACGACGC CACGGCGGAA    1860
GCGCCTTACG TGTTCAAACC TTCCACCGGC GATCTGATCA CCTTCGACGA TGCCCGCTCG    1920
GTGCAGGCTA AAGGCAAGTA CGTGTTGGAT AAGCAGCTGG GCGGCCTGTT CTCCTGGGAG    1980
ATCGACGCGG ATAACGGCGA TATTCTCAAC AGCATGAACG CCAGCCTGGG CAACAGCGCC    2040
GGCGTTCAAT AATCGGTTGC AGTGGTTGCC GGGGGATATC CTTTCGCCCC CGGCTTTTTC    2100
GCCGACGAAA GTTTTTTTAC GCCGCACAGA TTGTGGCTCT GCCCCGAGCA AAACGCGCTC    2160
ATCGGACTCA CCCTTTTGGG TAATCCTTCA GCATTTCCTC CTGTCTTTAA CGGCGATCAC    2220
AAAAATAACC GTTCAGATAT TCATCATTCA GCAACAAAGT TTTGGCGTTT TTTAACGGAG    2280
TTAAAAACCA GTAAGTTTGT GAGGGTCAGA CCAATGCGCT AAAAATGGG                2329
```

Sequenzprotokoll 9 und 10 (ChiG) :

Seq IDNO.: 9 und 10
Art der Sequenz: Nukleotid
Sequenzlänge: 1013 Nukleotide
Art des Molkeküls: cDNA
Ursprüngliche Herkunft: Gerstesamen (Hordeum vulgare L.)
Name: ChiG (Chitinase-G)
Merkmal:
63 pb-lange 5'-nicht übersetzende Anfangsregion, 798 pb offenes Ableseraster, 152 pb-langes 3'-nicht übersetztes Ende, Ablesestopcodons sind mit einem Sternchen markiert, die wahrscheinlichen Signalpeptidsequenzen sind unterstrichen, die abgeleitete Aminosäurensequenz eines 26 kD-Chitinase Präproteins mit 266 Aminosäuren ist unterhalb der Nukleotidsequenz angegeben, die unterstrichene AT-reiche Sequenz bei Position 905 ist wahrscheinlich ein Polyadenylierungssignal.
Eigenschaften:

Antifungal wirksam, insbesondere gegen Trichoderma reesii und fusarium sporotrichoides sowie Rhizoctonia solani und Botrytis cinerea.

SEQ ID NO: 9:

```
CCTACGACAG TAGCGTAACG GTAAACACCG AGTACGGTAC TCTGTGCTTT GTTGGCTCGC      60

ACA ATG AGA TCG CTC GCG GTG GTG GTG GCC GTG GTA GCC ACG GTG GCC       108
    Met Arg Ser Leu Ala Val Val Val Ala Val Val Ala Thr Val Ala
    -23         -20                 -15                 -10

ATG GCC ATC GGC ACG GCG CGC GGC AGC GTG TCC TCC ATC GTC TCG CGC       156
Met Ala Ile Gly Thr Ala Arg Gly Ser Val Ser Ser Ile Val Ser Arg
                -5                  1               5

GCA CAG TTT GAC CGC ATG CTT CTC CAC CGC AAC GAC GGC GCC TGC CAG       204
Ala Gln Phe Asp Arg Met Leu Leu His Arg Asn Asp Gly Ala Cys Gln
     10                 15                  20

GCC AAG GGC TTC TAC ACC TAC GAC GCC TTC GTC GCC GCC GCA GCC GCC       252
Ala Lys Gly Phe Tyr Thr Tyr Asp Ala Phe Val Ala Ala Ala Ala Ala
 25                  30                  35                  40

TTC CCG GGC TTC GGC ACC ACC GGC AGC GCC GAC GCC CAG AAG CGC GAG       300
Phe Pro Gly Phe Gly Thr Thr Gly Ser Ala Asp Ala Gln Lys Arg Glu
                 45                  50                  55
```

```
GTG GCC GCC TTC CTA GCA CAG ACC TCC CAC GAG ACC ACC GGC GGG TGG       348
Val Ala Ala Phe Leu Ala Gln Thr Ser His Glu Thr Thr Gly Gly Trp
            60                  65                  70

GCG ACT GCA CCG GAC GGG GCC TTC GCC TGG GGC TAC TGC TTC AAG CAG       396
Ala Thr Ala Pro Asp Gly Ala Phe Ala Trp Gly Tyr Cys Phe Lys Gln
        75                  80                  85

GAA CGT GGC GCC TCC TCC GAC TAC TGC ACC CCG AGC GCA CAA TGG CCG       444
Glu Arg Gly Ala Ser Ser Asp Tyr Cys Thr Pro Ser Ala Gln Trp Pro
    90                  95                  100

TGC GCC CCC GGG AAG CGC TAC TAC GGC CGC GGG CCA ATC CAG CTC TCC       492
Cys Ala Pro Gly Lys Arg Tyr Tyr Gly Arg Gly Pro Ile Gln Leu Ser
105                 110                 115                 120

CAC AAC TAC AAC TAT GGA CCT GCC GGC CGG GCC ATC GGG GTC GAT CTG       540
His Asn Tyr Asn Tyr Gly Pro Ala Gly Arg Ala Ile Gly Val Asp Leu
                125                 130                 135

CTG GCC AAC CCG GAC CTG GTG GCC ACG GAC GCC ACT GTG GGC TTT AAG       588
Leu Ala Asn Pro Asp Leu Val Ala Thr Asp Ala Thr Val Gly Phe Lys
            140                 145                 150

ACG GCC ATC TGG TTC TGG ATG ACG GCG CAG CCG CCC AAG CCA TCG AGC       636
Thr Ala Ile Trp Phe Trp Met Thr Ala Gln Pro Pro Lys Pro Ser Ser
        155                 160                 165

CAT GCT GTG ATC GCC GGC CAG TGG AGC CCG TCA GGG GCT GAC CGG GCC       684
His Ala Val Ile Ala Gly Gln Trp Ser Pro Ser Gly Ala Asp Arg Ala
    170                 175                 180

GCA GGC CGG GTG CCC GGG TTT GGT GTG ATC ACC AAC ATC ATC AAC GGC       732
Ala Gly Arg Val Pro Gly Phe Gly Val Ile Thr Asn Ile Ile Asn Gly
185                 190                 195                 200

GGG ATC GAG TGC GGT CAC GGG CAG GAC AGC CGC GTC GCC GAT CGA ATC       780
Gly Ile Glu Cys Gly His Gly Gln Asp Ser Arg Val Ala Asp Arg Ile
                205                 210                 215

GGG TTT TAC AAG CGC TAC TGT GAC ATC CTC GGC GTT GGC TAC GGC AAC       828
Gly Phe Tyr Lys Arg Tyr Cys Asp Ile Leu Gly Val Gly Tyr Gly Asn
            220                 225                 230

AAC CTC GAT TGC TAC AGC CAG AGA CCC TTC GCC TAATTAATTA GTCATGTATT     881
Asn Leu Asp Cys Tyr Ser Gln Arg Pro Phe Ala
        235                 240

AATCTTGGCC CTCCATAAAA TACAATAAGA GCATCGTCTC CTATCTACAT GCTGTAAGAT     941

GTAACTATGG TAACCTTTTA TGGGGAACAT AACAAAGGCA TCTCGTATAG ATGCTTTGCT    1001

A                                                                    1002
```

SEQ ID NO: 10 (Aminosäuresequenz der SEQ ID NO: 9)

```
Met Arg Ser Leu Ala Val Val Val Ala Val Val Ala Thr Val Ala Met
-23         -20                 -15                 -10

Ala Ile Gly Thr Ala Arg Gly Ser Val Ser Ser Ile Val Ser Arg Ala
        -5                  1               5

Gln Phe Asp Arg Met Leu Leu His Arg Asn Asp Gly Ala Cys Gln Ala
 10                  15                  20                  25

Lys Gly Phe Tyr Thr Tyr Asp Ala Phe Val Ala Ala Ala Ala Ala Phe
                30                  35                  40

Pro Gly Phe Gly Thr Thr Gly Ser Ala Asp Ala Gln Lys Arg Glu Val
            45                  50                  55

Ala Ala Phe Leu Ala Gln Thr Ser His Glu Thr Thr Gly Gly Trp Ala
        60                  65                  70

Thr Ala Pro Asp Gly Ala Phe Ala Trp Gly Tyr Cys Phe Lys Gln Glu
    75                  80                  85

Arg Gly Ala Ser Ser Asp Tyr Cys Thr Pro Ser Ala Gln Trp Pro Cys
 90                  95                 100                 105

Ala Pro Gly Lys Arg Tyr Tyr Gly Arg Gly Pro Ile Gln Leu Ser His
                110                 115                 120

Asn Tyr Asn Tyr Gly Pro Ala Gly Arg Ala Ile Gly Val Asp Leu Leu
            125                 130                 135

Ala Asn Pro Asp Leu Val Ala Thr Asp Ala Thr Val Gly Phe Lys Thr
        140                 145                 150

Ala Ile Trp Phe Trp Met Thr Ala Gln Pro Pro Lys Pro Ser Ser His
    155                 160                 165

Ala Val Ile Ala Gly Gln Trp Ser Pro Ser Gly Ala Asp Arg Ala Ala
170                 175                 180                 185

Gly Arg Val Pro Gly Phe Gly Val Ile Thr Asn Ile Ile Asn Gly Gly
                190                 195                 200

Ile Glu Cys Gly His Gly Gln Asp Ser Arg Val Ala Asp Arg Ile Gly
            205                 210                 215

Phe Tyr Lys Arg Tyr Cys Asp Ile Leu Gly Val Gly Tyr Gly Asn Asn
        220                 225                 230

Leu Asp Cys Tyr Ser Gln Arg Pro Phe Ala
    235                 240
```

Sequenzprotokolle 11 und 12 (GluG):

Seq IDNo.: 11 und 12
Art der Sequenz: Nukleotid mit entsprechendem Protein
Sequenzlänge: 1249 Nukleotide
Art des Moleküls: cDNA

Ursprüngliche Herkunft: Gerstesamen (Hordeum vulgare L.)
Name: GluG (Glukanase)
Merkmale: 48 bp-lange 5'-nicht übersetzende Anfangsregion Offenes Ableseraster von 1002 bp
199 pb-langes 3'-nicht übersetztes Ende,
die unterstrichene At-reiche Sequenz bei den Position 1083 und 1210 sind wahrscheinlich Polyadenylierungssignale, die abgeleitete Aminosäuresequenz des codierten Präproteins von 334 Aminosäuren wird unterhalb der Nukleotidsequenz angegeben.

```
                                      TACTCCGTGT GTGCACCA ATG GCT AGA      57
                                                          Met Ala Arg
                                                          -28

AAA GAT GTT GCC TCC ATG TTT GCA GTT GCT CTC TTC ATT GGA GCA TTC     105
Lys Asp Val Ala Ser Met Phe Ala Val Ala Leu Phe Ile Gly Ala Phe
-25                 -20                 -15                 -10

GCT GCT GTT CCT ACG AGT GTG CAG TCC ATC GGC GTA TGC TAC GGC GTG     153
Ala Ala Val Pro Thr Ser Val Gln Ser Ile Gly Val Cys Tyr Gly Val
                -5                  1               5

ATC GGC AAC AAC CTC CCC TCC CGG AGC GAC GTG GTG CAG CTC TAC AGG     201
Ile Gly Asn Asn Leu Pro Ser Arg Ser Asp Val Val Gln Leu Tyr Arg
        10                  15                  20

TCC AAG GGC ATC AAC GGC ATG CGC ATC TAC TTC GCC GAC GGG CAG GCC     249
Ser Lys Gly Ile Asn Gly Met Arg Ile Tyr Phe Ala Asp Gly Gln Ala
    25                  30                  35
```

```
CTC TCG GCC GTC CGC AAC TCC GGC ATC GGC CTC ATC CTC GAC ATC GGC     297
Leu Ser Ala Val Arg Asn Ser Gly Ile Gly Leu Ile Leu Asp Ile Gly
 40                  45                  50                  55

AAC GAC CAG CTC GCC AAC ATC GCC GCC AGC ACC TCC AAC GCG GCC TCC     345
Asn Asp Gln Leu Ala Asn Ile Ala Ala Ser Thr Ser Asn Ala Ala Ser
                 60                  65                  70

TGG GTC CAG AAC AAC GTG CGG CCC TAC TAC CCT GCC GTG AAC ATC AAG     393
Trp Val Gln Asn Asn Val Arg Pro Tyr Tyr Pro Ala Val Asn Ile Lys
             75                  80                  85

TAC ATC GCC GCC GGC AAC GAG GTG CAG GGC GGC GCC ACG CAG AGC ATC     441
Tyr Ile Ala Ala Gly Asn Glu Val Gln Gly Gly Ala Thr Gln Ser Ile
         90                  95                 100

CTG CCG GCC ATG CGC AAC CTC AAC GCG GCC CTC TCC GCG GCG GGG CTC     489
Leu Pro Ala Met Arg Asn Leu Asn Ala Ala Leu Ser Ala Ala Gly Leu
    105                 110                 115

GGC GCC ATC AAG GTG TCC ACC TCC ATC CGG TTC GAC GAG GTG GCC AAC     537
Gly Ala Ile Lys Val Ser Thr Ser Ile Arg Phe Asp Glu Val Ala Asn
120                 125                 130                 135

TCC TTC CCG CCC TCC GCC GGC GTG TTC AAG AAC GCC TAC ATG ACG GAC     585
Ser Phe Pro Pro Ser Ala Gly Val Phe Lys Asn Ala Tyr Met Thr Asp
                140                 145                 150

GTG GCC CGG CTC CTG GCG AGC ACC GGC GCG CCG CTG CTC GCC AAC GTC     633
Val Ala Arg Leu Leu Ala Ser Thr Gly Ala Pro Leu Leu Ala Asn Val
            155                 160                 165

TAC CCC TAC TTC GCG TAC CGT GAC AAC CCC GGG AGC ATC AGC CTG AAC     681
Tyr Pro Tyr Phe Ala Tyr Arg Asp Asn Pro Gly Ser Ile Ser Leu Asn
        170                 175                 180

TAC GCG ACG TTC CAG CCG GGC ACC ACC GTG CGT GAC CAG AAC AAC GGG     729
Tyr Ala Thr Phe Gln Pro Gly Thr Thr Val Arg Asp Gln Asn Asn Gly
    185                 190                 195

CTG ACC TAC ACG TCC CTG TTC GAC GCG ATG GTG GAC GCC GTG TAC GCG     777
Leu Thr Tyr Thr Ser Leu Phe Asp Ala Met Val Asp Ala Val Tyr Ala
200                 205                 210                 215

GCG CTG GAG AAG GCC GGC GCG CCG GCG GTG AAG GTG GTG GTG TCG GAG     825
Ala Leu Glu Lys Ala Gly Ala Pro Ala Val Lys Val Val Val Ser Glu
                220                 225                 230

AGC GGG TGG CCG TCG GCG GGC GGG TTT GCG GCG TCG GCC GGC AAT GCG     873
Ser Gly Trp Pro Ser Ala Gly Gly Phe Ala Ala Ser Ala Gly Asn Ala
            235                 240                 245

CGG ACG TAC AAC CAG GGG CTG ATC AAC CAC GTC GGC GGG GGC ACG CCC     921
Arg Thr Tyr Asn Gln Gly Leu Ile Asn His Val Gly Gly Gly Thr Pro
        250                 255                 260

AAG AAG CGG GAG GCG CTG GAG ACG TAC ATC TTC GCC ATG TTC AAC GAG     969
Lys Lys Arg Glu Ala Leu Glu Thr Tyr Ile Phe Ala Met Phe Asn Glu
    265                 270                 275
```

SEQ ID NO: 12 (Aminosäuresequant der SEQ ID NO: 11)

```
Met Ala Arg Lys Asp Val Ala Ser Met Phe Ala Val Ala Leu Phe Ile
-28         -25                 -20                 -15

Gly Ala Phe Ala Ala Val Pro Thr Ser Val Gln Ser Ile Gly Val Cys
        -10                 -5                  1

Tyr Gly Val Ile Gly Asn Asn Leu Pro Ser Arg Ser Asp Val Val Gln
 5                  10                  15                  20

Leu Tyr Arg Ser Lys Gly Ile Asn Gly Met Arg Ile Tyr Phe Ala Asp
                25                  30                  35

Gly Gln Ala Leu Ser Ala Val Arg Asn Ser Gly Ile Gly Leu Ile Leu
            40                  45                  50

Asp Ile Gly Asn Asp Gln Leu Ala Asn Ile Ala Ala Ser Thr Ser Asn
        55                  60                  65

Ala Ala Ser Trp Val Gln Asn Asn Val Arg Pro Tyr Tyr Pro Ala Val
    70                  75                  80

Asn Ile Lys Tyr Ile Ala Ala Gly Asn Glu Val Gln Gly Gly Ala Thr
 85                 90                  95                  100

Gln Ser Ile Leu Pro Ala Met Arg Asn Leu Asn Ala Ala Leu Ser Ala
                105                 110                 115

Ala Gly Leu Gly Ala Ile Lys Val Ser Thr Ser Ile Arg Phe Asp Glu
            120                 125                 130

Val Ala Asn Ser Phe Pro Pro Ser Ala Gly Val Phe Lys Asn Ala Tyr
        135                 140                 145

Met Thr Asp Val Ala Arg Leu Leu Ala Ser Thr Gly Ala Pro Leu Leu
    150                 155                 160

Ala Asn Val Tyr Pro Tyr Phe Ala Tyr Arg Asp Asn Pro Gly Ser Ile
165                 170                 175                 180

Ser Leu Asn Tyr Ala Thr Phe Gln Pro Gly Thr Thr Val Arg Asp Gln
                185                 190                 195

Asn Asn Gly Leu Thr Tyr Thr Ser Leu Phe Asp Ala Met Val Asp Ala
            200                 205                 210

Val Tyr Ala Ala Leu Glu Lys Ala Gly Ala Pro Ala Val Lys Val Val
        215                 220                 225

Val Ser Glu Ser Gly Trp Pro Ser Ala Gly Gly Phe Ala Ala Ser Ala
    230                 235                 240

Gly Asn Ala Arg Thr Tyr Asn Gln Gly Leu Ile Asn His Val Gly Gly
245                 250                 255                 260

Gly Thr Pro Lys Lys Arg Glu Ala Leu Glu Thr Tyr Ile Phe Ala Met
                265                 270                 275

Phe Asn Glu Asn Gln Lys Thr Gly Asp Ala Thr Glu Arg Ser Phe Gly
            280                 285                 290

Leu Phe Asn Pro Asp Lys Ser Pro Ala Tyr Asn Ile Gln Phe
        295                 300                 305
```

## Patentansprüche

1. Transgene Pilz-resistente Pflanze, dessen Genom mindestens zwei unterschiedliche Gen-Sequenzen, die für Proteine mit Pilz-inhibierender Wirkung kodieren, unter Kontrolle aktiver Promotoren, umfaßt, **dadurch gekennzeichnet, daß** die DNA-Sequenzen kodieren für:

   (a) mindestens ein PSI-Protein umfassend die Aminosäuresequenz gemäß Sequenz ID NO. 4, und
   (b) ein ChiS-Protein umfassend die Aminosäuresequenz des Genprodukts der Sequenz gemäß Sequenz ID NO. 8 oder ein AFP-Protein umfassend die Aminosäuresequenz gemäß Sequenz ID NO. 3.

2. Pilz-resistente Pflanze gemäß Anspruch 1, wobei die Pflanze dadurch erhalten wurde, daß in das Genom einer Pflanze mindestens ein Gen, das für ein Protein mit Pilz-inhibierender Wirkung kodiert, transferiert und die Pilz-resistente Pflanze

   (a) durch Kreuzung der Pflanze mit einer gegebenenfalls transgenen, weiteren Pflanze, die mindestens ein anderes Gen, das für ein anderes Protein mit Pilz-inhibierender Wirkung kodiert, enthält und anschließende Selektion oder
   (b) durch Transformation der Pflanze mit mindestens einem anderen Gen, das für ein anderes Protein mit Pilz-inhibierender Wirkung kodiert, erhalten wird.

3. Pflanze nach Anspruch 1 oder 2, **dadurch gekennzeichnet, daß** die Pflanze eine Tabak-, Kartoffel-, Erdbeer-, Mais-, Raps- oder Tomatenpflanze ist.

4. Verfahren zur Erzeugung Pilz-resistenter Pflanzen nach einem der Ansprüche 1 bis 3, **dadurch gekennzeichnet, daß** in das Genom einer Pflanze mindestens ein Gen, das für ein Protein mit Pilz-inhibierender Wirkung wie in Anspruch 1 definiert kodiert, transferiert, und die Pilz-resistente Pflanze

   (a)' durch Kreuzung der Pflanze mit einer gegebenenfalls transgenen weiteren Pflanze, die mindestens ein anderes Gen, das für ein anderes Protein mit Pilz-inhibierender Wirkung wie in Anspruch 1 definiert kodiert, enthält und anschließende Selektion oder
   (b)' durch Transformation der Pflanze mit mindestens einem anderen Gen, das für ein anderes Protein mit Pilz-inhibierender Wirkung wie in Anspruch 1 definiert kodiert, erhalten wird.

5. Verfahren zur Erzeugung einer Pflanze mit verbesserter Pilzresistenz, wobei das Verfahren die Transformation einer Pflanze mit mindestens zwei unterschiedlichen Gen-Sequenzen, die für Proteine mit Pilz-inhibierender Wirkung kodieren, unter Kontrolle aktiver Promotoren, umfaßt, mit der Maßgabe, daß die DNA-Sequenzen kodieren für:

   (a) mindestens ein PSI-Protein umfassend die Aminosäuresequenz gemäß Sequenz ID NO. 4, und
   (b) ein ChiS-Protein umfassend die Aminosäuresequenz des Genprodukts der Sequenz gemäß Sequenz ID NO. 8 oder ein AFP-Protein umfassend die Aminosäuresequenz gemäß Sequenz ID NO. 3.

## Claims

1. Transgenic, fungus-resistant plant whose genome comprises at least two different gene sequences which code for proteins with fungus-inhibiting effects under the control of active promoters, **characterized by** DNA sequences coding for:

   (a) at least one PSI protein comprising the amino acid sequence according to the sequence ID No. 4, and

   (b) a ChiS protein comprising the amino acid sequence of the gene product of the sequence according to sequence ID No. 8 or a AFP protein comprising the amino acid sequence according to sequence ID No. 3.

2. Fungus-resistant plant according to claim 1 and said plant obtained by at least one gene being transferred into the genome of a plant which codes for a protein with a fungus-inhibiting effect and the fungus-resistant plant being obtained by

(a) crossing of said plant with another optionally transgenic plant which contains at least another gene which codes for another gene with a fungus-inhibiting effect, followed by selection or

(b) transformation of said plant with at least another gene which codes for another protein with a fungus-inhibiting effect.

**3.** Said plant according to claims 1 or 2 being a tobacco, potato, strawberry, corn, rape or tomato plant.

**4.** A process for generation of fungus-resistant plants according to any of claims 1 to 3, **characterized by** at least one gene, which codes for a protein, with a fungus-inhibiting effect according to claim 1, being transferred into the genome of one plant and the fungus-resistant plant being obtained

(a)' by crossing of said plant with another, optionally transgenic, plant comprising at least another gene which codes for another protein, with a fungus-inhibiting effect, as defined in claim 1, followed by selection, or

(b)' by transformation of said plant with at least another gene, which codes for another protein, with a fungus-resistant effect according to claim 1.

**5.** A process for generation of a plant with improved fungus-resistance wherein said process comprises transformation of a plant with at least two different gene sequences, coding for proteins with a fungus-inhibiting effect with the proviso that the DNA sequences code for:

(a) at least one PSI protein comprising the amino acid sequence according to the sequence ID No. 4, and

(b) a ChiS protein comprising the amino acid sequence of the gene product of the sequence according to sequence ID No. 8 or a AFP protein comprising the amino acid sequence according to sequence ID No. 3.

**Revendications**

**1.** Plante transgénique résistante aux champignons dont le génome, sous le contrôle de promoteurs actifs, comprend au moins deux différentes séquences de gènes codant pour des protéines à effet antifongique, **caractérisée en ce que** les séquences d'ADN codent pour :

(a) au moins une protéine PSI comprenant la séquence d'acides aminés selon la séquence ID n° 4, et
(b) une protéine ChiS comprenant la séquence d'acides aminés du produit du gène de la séquence selon la séquence ID n° 8 ou une protéine AFP comprenant la séquence d'acides aminés selon la séquence ID n° 3.

**2.** Plante résistante aux champignons selon la revendication 1, la plante ayant été obtenue par le transfert dans le génome d'une plante d'au moins un gène codant pour une protéine à effet antifongique et la plante résistante aux champignons étant obtenue

(a) par croisement de la plante avec une autre plante éventuellement transgénique laquelle contient au moins un autre gène codant pour une autre protéine à effet antifongique, et ensuite par sélection ou
(b) par transformation de la plante avec au moins un autre gène codant pour une autre protéine à effet antifongique.

**3.** Plante selon revendication 1 ou 2, **caractérisée en ce que** la plante est un plant de tabac, de pomme de terre, de fraisier, de maïs, de colza ou de tomate.

**4.** Procédé pour la production de plantes résistantes aux champignons selon l'une des revendications 1 à 3, **caractérisé en ce qu'**au moins un gène codant pour une protéine à effet antifongique tel que défini dans la revendication 1 est transféré dans le génome d'une plante et que la plante résistante aux champignons est obtenue

(a)' par croisement de la plante avec une autre plante éventuellement transgénique laquelle contient au moins un autre gène codant pour une autre protéine à effet antifongique tel que défini dans la revendication 1, et ensuite par sélection ou
(b)' par transformation de la plante avec au moins un autre gène codant pour une autre protéine à effet antifongique tel que défini dans la revendication 1.

**5.** Procédé pour la production d'une plante à résistance fongique améliorée, le procédé comprenant, sous le contrôle de promoteurs actifs, la transformation d'une plante avec au moins deux différentes séquences de gènes codant pour des protéines à effet antifongique, afin que les séquences d'ADN codent pour :

(a) au moins une protéine PSI comprenant la séquence d'acides aminés selon la séquence ID n° 4, et

(b) une protéine ChiS comprenant la séquence d'acides aminés du produit du gène de la séquence selon la séquence ID n° 8 ou une protéine AFP comprenant la séquence d'acides aminés selon la séquence ID n° 3.

Fig. 1

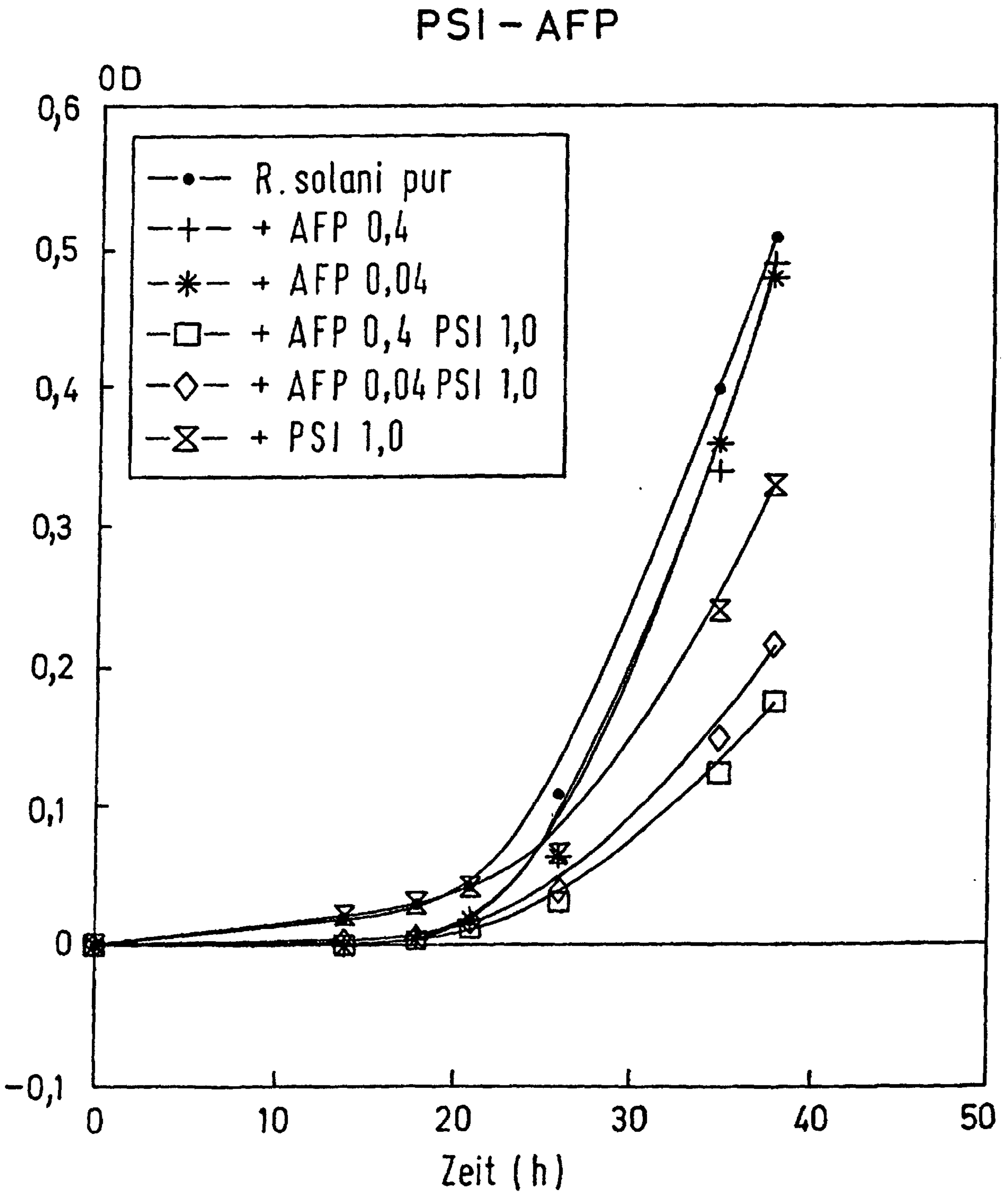
PSI - AFP
OD
0,6
0,5
0,4
0,3
0,2
0,1
0
-0,1
R. solani pur
+ AFP 0,4
+ AFP 0,04
+ AFP 0,4 PSI 1,0
+ AFP 0,04 PSI 1,0
+ PSI 1,0
0
10
20
30
40
50
Zeit (h)

Fig. 2

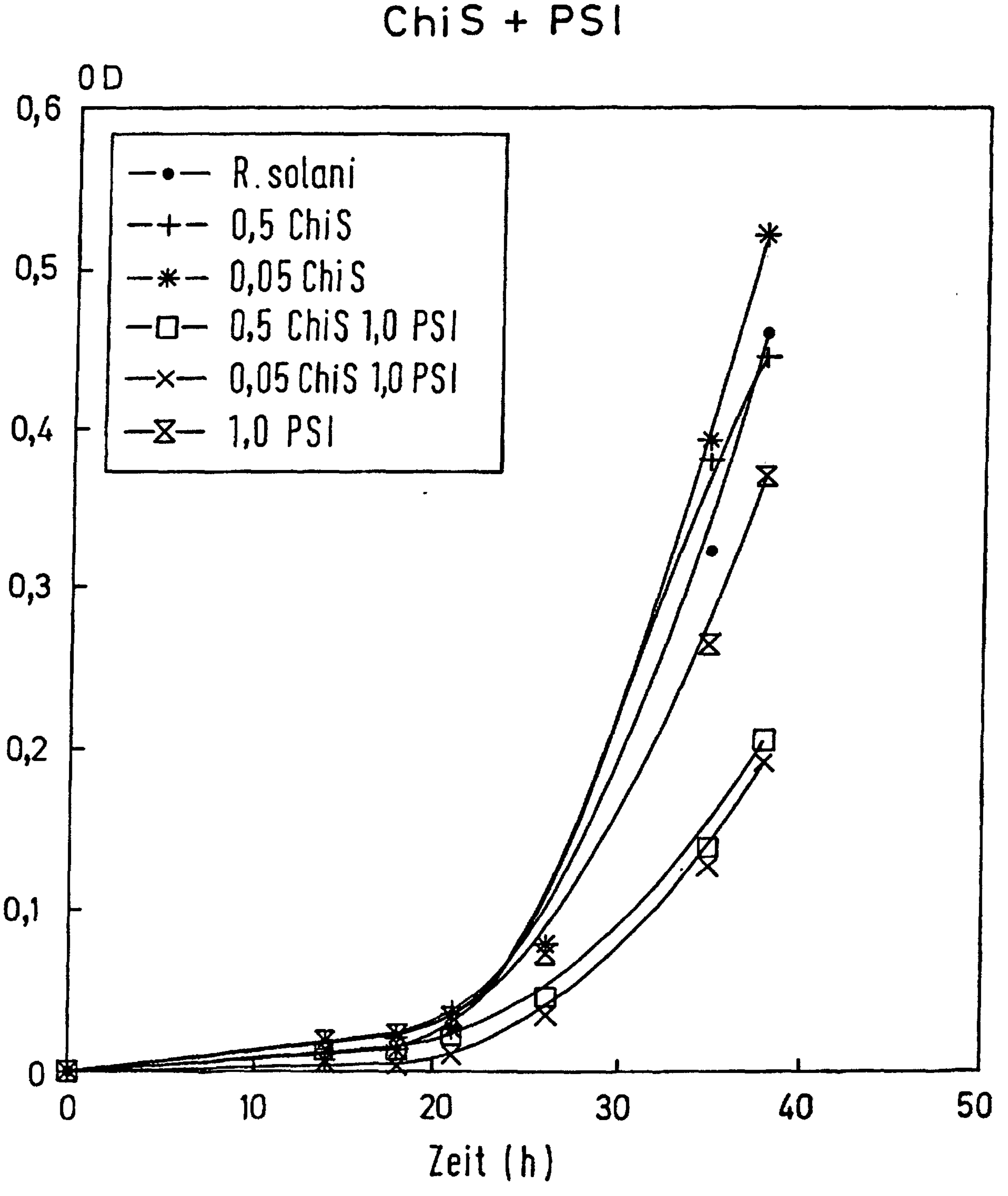
ChiS + PSI
OD
0,6
0,5
0,4
0,3
0,2
0,1
0
R.solani
0,5 ChiS
0,05 ChiS
0,5 ChiS 1,0 PSI
0,05 ChiS 1,0 PSI
1,0 PSI
0
10
20
30
40
50
Zeit (h)